# EUROPEAN PATENT APPLICATION

(11) **EP 2 902 958 A1**
(43) Date of publication of application: **05.08.2015**
(21) Application number: 13841871.0
(22) Date of filing: 26.08.2013
(51) Int. Cl.: G06Q 50/22, G06Q 40/08, G06Q 30/02, H04L 12/28, G06F 19/00, G09B 19/00

(54) **METHOD FOR SPECIFYING BEHAVIOR TRENDS AND SYSTEM FOR SPECIFYING BEHAVIOR TRENDS**

(30) Priority: 28.09.2012 JP 2012217907; 05.10.2012 US 201261710025 P
(71) Applicant: Panasonic Intellectual Property Corporation of America, Torrance, CA 90503 (US)
(72) Inventor: SAKATA, Kotaro, Osaka 540-6207 (JP); MARUYAMA, Tomoaki, Osaka 540-6207 (JP); KONDO, Kenji, Osaka 540-6207 (JP); TOJIMA, Masayoshi, Osaka 540-6207 (JP); YAMAMOTO, Hiroaki, Osaka 540-6207 (JP)
(74) Representative: Vigand, Philippe
(86) International application number: PCT/JP2013/005029
(87) International publication number: WO 2014/049953

(57) **Abstract**

There are included: a step (S501) for receiving, through communication, pieces of operation information each including (a) operating state information of a device and (b) user information for identifying a user, in association with each other; a step (S502) storing the pieces of the operation information received in the receiving (S501) into a first storage unit (202); a step (S503) for reading, from a second storage unit, behavior tendency information including, in association with each other, (c) operating state information of at least one device and (d) a predetermined user behavior tendency of the user; and a step (S504) for specifying the predetermined user behavior tendency which corresponds to the operating state information of at least one device, by searching the pieces of the operation information for the operating state information of at least one device which is included in the behavior tendency information.

## Description

### [Technical Field]

The present invention relates to methods of integrating pieces of operation information obtained from a plurality of devices and specifying a tendency of user's behavior.

### [Background Art]

Conventionally, there has been known a server which obtains pieces of information related to exercises and eating and drinking, and sends, to a user's mobile phone, an electronic mail as an advice or an alarm in which the pieces of information are integrated (see

Patent Literature 1).

There has been also known a technique of calculating, based on biological information associated with identification information of a subject, a probability that a subject belongs to a progression stage class for each predetermined disease to be prevented and ameliorated (see Patent Literature 2).

### [Citation List]

### [Patent Literature]

[PTL 1] Japanese Unexamined Patent Application Publication No. 2002-24404
[PTL 2] Japanese Unexamined Patent Application Publication No. 2010-231308

### [Summary of Invention]

### [Technical Problem]

However, the conventional techniques have difficulties of specifying a tendency of user's behavior from multilateral viewpoints based on user's daily behavior.

Thus, the present invention overcomes the problems of the conventional techniques. It is an object of the present invention to provide a method and a system for specifying a tendency of user's behavior from multilateral viewpoints based on user's daily behavior.

### [Solution to Problem]

In accordance with an aspect of the present invention for achieving the object, there is provided a method for specifying a behavior tendency, the method comprising: receiving, through communication, pieces of operation information each including (a) operating state information of a corresponding one of devices and (b) user information for identifying a user, in association with each other; storing the pieces of the operation information received in the receiving into a first storage unit; reading at least one piece of behavior tendency information from a second storage unit, the at least one piece of the behavior tendency information each including, in association with each other, (c) operating state information of at least one of the devices and (d) a predetermined user behavior tendency of the user; and specifying, based on the at least one piece of the behavior tendency information read in the reading and the pieces of the operation information stored in the first storage unit, the predetermined user behavior tendency which corresponds to the operating state information of the at least one of the devices, by searching the pieces of the operation information for the operating state information of the at least one of the devices which is included in the at least one piece of the behavior tendency information.

These general and specific aspects may be implemented to a system, a method, an integrated circuit, a computer program, and a computer-readable recording medium, such as a Compact Disc-Read Only Memory (CD-ROM), and may be implemented also to a desired combination of them.

### [Advantageous Effects of Invention]

The method for specifying a behavior tendency and a system for specifying the behavior tendency according to the present invention are capable of specifying a tendency of user's behavior from multilateral viewpoints based on user's daily behavior.

### [Brief Description of Drawings]

[FIG. 1] FIG. 1 is a diagram illustrating a configuration of a system according to Embodiment 1 of the present invention.
[FIG. 2] FIG. 2 is a diagram illustrating a structure of a server device according to Embodiment 1 of the present invention.
[FIG. 3A] FIG. 3A is an example of data stored in a first storage unit according to Embodiment 1 of the present invention.
[FIG. 3B] FIG. 3B is an example of data stored in the first storage unit according to Embodiment 1 of the present invention.
[FIG. 4A] FIG. 4A is an example of data stored in a second storage unit according to Embodiment 1 of the present invention.
[FIG. 4B] FIG. 4B plots an example of data stored in the second storage unit and an example of user's actual data according to Embodiment 1 of the present invention.
[FIG. 4C] FIG. 4C plots an example of data stored in the second storage unit and an example of user's actual data according to Embodiment 1 of the present invention.
[FIG. 5] FIG. 5 is a flowchart of a specification unit according to Embodiment 1 of the present invention.
[FIG. 6] FIG. 6 is a diagram illustrating a structure of a server device according to Embodiment 2 of the present invention.
[FIG. 7] FIG. 7 is a flowchart of an adjustment unit according to Embodiment 2 of the present invention.
[FIG. 8] FIG. 8 is a diagram illustrating a structure of a server device according to Embodiment 3 of the present invention.
[FIG. 9A] FIG. 9A is a flowchart of a compensation calculation unit according to Embodiment 3 of the present invention.
[FIG. 9B] FIG. 9B is a flowchart of a compensation calculation unit according to Embodiment 3 of the present invention.

### [Description of Embodiments]

### (Observation based on which Present Disclosure is Conceived)

The inventors of the present invention have found the following problems in the server and the like disclosed in "Background Art".

In Patent Literature 1, more specifically, a user's exercise amount is automatically detected by an exercise amount measuring device or a mobile phone having a Global Positioning System (GPS) function, and a user's eating/drinking state is obtained from a cash register in which nutrient information is managed for each menu. Both the exercise amount and the eating/drinking state are registered in a server via the Internet. The server generates an advice in which the exercise amount and the eating/drinking state are integrated.

In Patent Literature 1, however, since eating/drinking information is obtained from a cash register, it is difficult to manage information indicating when a user actually eats and drinks, although it is possible to manage information of nutrients of foods and drinks which the user has bought or has taken. Furthermore, if the cash register does not correspond to the information, it is impossible to obtain the information. Therefore, the user needs to input the information by an information terminal such as a personal computer and transmits the information to the server.

Moreover, in Patent Literature 2, a user needs to do bothersome operation of inputting biological information to a user's terminal device.

As described above, the conventional techniques have the difficulties in specifying a tendency of user's behavior from multilateral viewpoints without causing the user to do bothersome operation of inputting information. In particular, the conventional techniques have difficulties of detecting user's daily behavior in home without using a dedicated measuring device, in order to specify a user behavior tendency.

In order to solve the above conventional problems, in accordance with an aspect of the present invention, there is provided a method for specifying a behavior tendency, the method comprising: receiving, through communication, pieces of operation information each including (a) operating state information of a corresponding one of devices and (b) user information for identifying a user, in association with each other; storing the pieces of the operation information received in the receiving into a first storage unit; reading at least one piece of behavior tendency information from a second storage unit, the at least one piece of the behavior tendency information each including, in association with each other, (c) operating state information of at least one of the devices and (d) a predetermined user behavior tendency of the user; and specifying, based on the at least one piece of the behavior tendency information read in the reading and the pieces of the operation information stored in the first storage unit, the predetermined user behavior tendency which corresponds to the operating state information of the at least one of the devices, by searching the pieces of the operation information for the operating state information of the at least one of the devices which is included in the at least one piece of the behavior tendency information.

By this, user's daily life behavior can be specified from operating states of the devices including home appliances. Therefore, it is possible to specify a user behavior tendency from multilateral viewpoints.

Furthermore, for example, it is possible that each of the pieces of the operation information includes the operating state information that is generated at a corresponding time for the corresponding one of the devices, and in the specifying, the pieces of the operation information stored in the first storage unit are compared to the at least one piece of the behavior tendency information to determine whether or not behavior of the user is performed at an appropriate time, and the predetermined user behavior tendency is specified based on a result of the determining.

By this, it is possible to specify a user behavior tendency based on timeliness of behavior, such as a bedtime and a awake time, in other words, whether or not the user goes to bed early and gets up early, whether a time zone for meal is not in a desired time zone, whether or not the time zone is biased, or whether or not the user often has supper.

It is also possible, for example, that each of the pieces of the operation information includes the operating state information that is generated at a corresponding time for the corresponding one of the devices, and in the specifying, the pieces of the operation information stored in the first storage unit are compared to the at least one piece of the behavior tendency information to determine whether or not behavior of the user is performed regularly, and the predetermined user behavior tendency is specified based on a result of the determining.

By this, it is possible to specify a user behavior tendency based on the regularity of behavior, for example, based on regularity of behavior, such as whether or not a sleeping time is regular, or whether or not house cleaning or brushing teeth is performed in an appropriate cyclic way and at an appropriate frequency.

It is further possible, for example, that in the reading, the at least one piece of the behavior tendency information includes an index that indicates the predetermined user behavior tendency and that is associated with each kind of the operating state information to increase or decrease an assessment value related to the user, and in the specifying, the predetermined user behavior tendency is specified, by comparing the pieces of the operation information stored in the first storage unit to the at least one piece of the behavior tendency information to calculate the assessment value.

By this, it is possible to integrate various kinds of user's behavior specified based on the operating state information of at least one of the devices, into a single assessment value of a user behavior tendency.

It is still further possible, for example, that the method further comprises adjusting the assessment value based on at least one of (a) a total number of the devices providing the pieces of the operation information associated with the user information and (b) types of the devices.

By this, it is possible to adjust a result of the analysis of a user behavior tendency, according to the number or types of devices regarding which the user provides information.

It is still further possible, for example, that in the adjusting, the assessment value of the user which is calculated in the specifying is adjusted to be lower as the total number of the devices providing the pieces of the operation information associated with the user information is greater.

By this, it is possible to give the user incentive to provide more pieces of information. As a result, is possible to induce the user to purchase the devices.

It is still further possible, for example, that in the adjusting, the assessment value of the user which is calculated in the specifying is adjusted to be lower as the pieces of the operation information associated with the user information include more pieces of information having a high importance level. It is still further possible, for example, that a kind of the information having the high importance level is urine data or blood sugar level data.

By this, it is possible to provide the user with incentive to provide information having a higher importance level. As a result, it is possible to induce the user to purchase the devices capable of providing information having a high importance level.

It is still further possible, for example, that the index in the at least one piece of the behavior tendency information is for calculating a risk of a disease of the user, and in the specifying, the predetermined user behavior tendency is specified, by comparing the pieces of the operation information stored in the first storage unit to the at least one piece of the behavior tendency information to calculate the risk. It is still further possible, for example, that the disease is a lifestyle-related disease.

By this, it is possible to calculate a risk of causing a disease which is influenced by daily life behavior. In particular, daily lifestyle habits are large factors of causing lifestyle-related diseases. Therefore, it is possible to calculate a risk of a lifestyle-related disease with high accuracy.

It is still further possible, for example, that the devices include a refrigerator, and the index in the at least one piece of the behavior tendency information is for calculating the risk of the user to be higher as the refrigerator is opened and closed at a higher frequency at night or before a bedtime.

By this, it is possible to calculate a risk influenced by a diet habit which is detected from, for example, a frequency of user's eating supper.

It is still further possible, for example, that the devices include a cleaner, and the index in the at least one piece of the behavior tendency information is for calculating the risk of the user to be higher as the cleaner is operated at least one of at a lower frequency and in a shorter time period.

By this, it is possible to calculate a risk influenced by a hygienic state of a space which is detected from, for example, a frequency of user's cleaning.

It is still further possible, for example, that the devices include an electric toothbrush, and the index in the at least one piece of the behavior tendency information is for calculating the risk of the user to be higher as the electric toothbrush is used at least one of at a lower frequency and in a shorter time period.

By this, it is possible to calculate a risk influenced by a hygienic habit which is detected from a frequency of user's tooth brushing or the like.

It is still further possible, for example, that the devices include an air cleaner, and the index in the at least one piece of the behavior tendency information is for calculating the risk of the user to be higher as a dust sensor in the air cleaner shows a greater value.

By this, it is possible to calculate a risk influenced by an air quality state of the space.

It is still further possible, for example, that the method further comprising calculating an economic compensation for the assessment value calculated in the specifying, based on the assessment value. It is still further possible, for example, that in the calculating of the economic compensation, a point to be given to the user is calculated as the economic compensation. It is still further possible, for example, in the calculating of the economic compensation, an insurance fee of insurance for the user is calculated as the economic compensation.

By this, the economic compensation is calculated based on the desirability of the daily life behavior. As a result, it is possible to provide the user with incentive to improve life behavior.

These general and specific aspects may be implemented to a system, a method, an integrated circuit, a computer program, and a computer-readable recording medium, such as a Compact Disc-Read Only Memory (CD-ROM), and may be implemented also to a desired combination of them.

The following describes a method for specifying a behavior tendency and a system for specifying the behavior tendency according to the aspects of the present invention in more detail with reference to the drawings.

### (Embodiment 1)

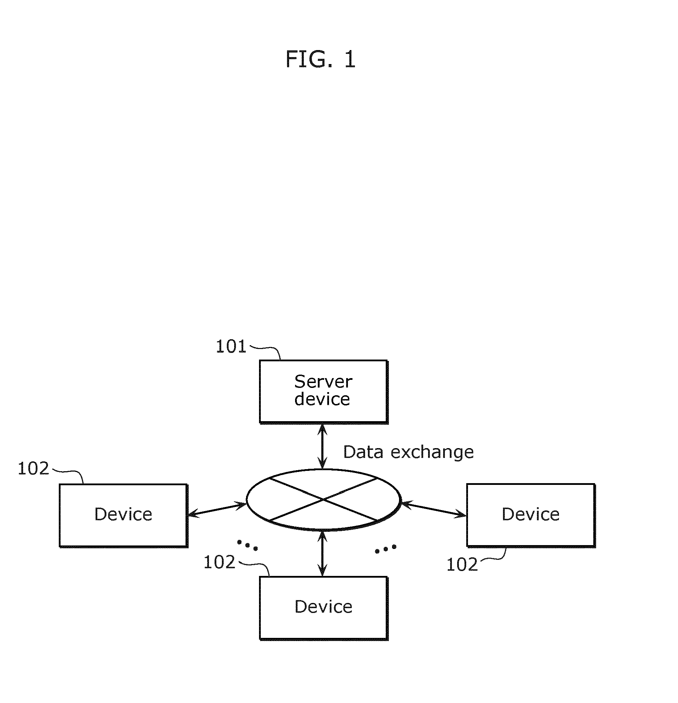

FIG. 1 is a diagram illustrating a configuration of a system according to Embodiment 1 of the present invention.

As illustrated in FIG. 1, the device 102, which has a function of being connected to a network, is connected to an external server device 101 via the network so as to exchange a program, data related to a user, control data for controlling the device, and the like between the device 102 and the server device 101.

The device may be any device or system. Examples of the device include: home appliances, such as a television set, an air conditioner, a refrigerator, a washing machine, a cleaner, an air cleaner, an electric toothbrush, and a dryer; information terminals, such as a personal computer, a mobile telephone, a smartphone, and a tablet; sensor devices, such as a blood sugar level sensor device; a toilet: a bathroom; a mirror; a lighting device; and the like.

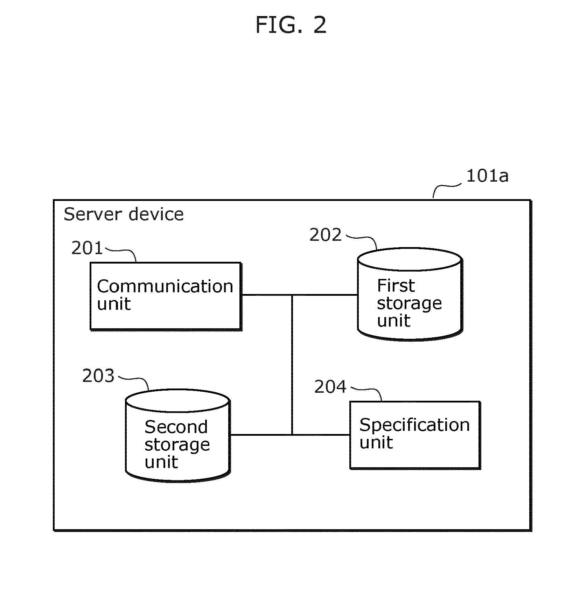

FIG. 2 is a diagram illustrating a structure of a server device according to Embodiment 1 of the present invention.

As illustrated in FIG. 2, a server device 101a includes a communication unit 201, a first storage unit 202, a second storage unit 203, and a specification unit 204. The server device 101a is an aspect of the server device 101 illustrated in FIG. 1.

More specifically, the server device 101a includes a microprocessor, a Read-Only Memory (ROM), a Random Access Memory (RAM), a hard disk, and the like which are not illustrated in the figure. In each of the ROM, the RAM, and the hard disk, a computer program is recorded. When the microprocessor operates according to the computer program, the server device 101a functions.

It should be noted that each functional block, such as the communication unit 201, the first storage unit 202, the second storage unit 203, or the specification unit 204, may be implemented to software, to a combination of the software and a Large Scale Integration (LSI) that is an integrated circuit (namely, hardware), or to the LSI (namely, hardware).

### (1) Communication Unit

The communication unit 201 receives, from each device, (a) change information indicating changes in an operating state of the device and (b) user information associated with the change information.

Here, as described above, the device is any device or system set in each home. Examples of the device include: home appliances, such as a television set, an air conditioner, a refrigerator, a washing machine, a cleaner, an air cleaner, an electric toothbrush, and a dryer; information terminals, such as a personal computer, a mobile telephone, a smartphone, and a tablet; sensor devices, such as a blood sugar level sensor device; a toilet: a bathroom; a mirror; a lighting device; and the like.

The user information includes an identifier (hereinafter, referred to as a "usher ID") for uniquely identifying a corresponding user. The user ID may be assigned to an individual user, or to a group of users using a corresponding device. More specifically, for example, the user ID may be assigned to family or people living together in the same home. The user ID is recorded offline or online onto a control unit of a device at an appropriate time such as a time of selling the device.

Each of devices is allocated with an identifier unique to the device (hereinafter, referred to as a "device ID"). For each of the devices, such a device ID is recorded on a control unit in the corresponding device when the device is manufactured. In addition, a control unit in each device manages a device state indicating an operating state of the device. The device state is changed by change of a state of a processing unit in the device. For example, the device state is changed when an input unit in the device receives an instruction of a new operation, when a detection result of a sensor in the device is changed to a predetermined result, or when it is a predetermined time. It should be noted that it has been described that a device ID is recorded on a control unit in manufacturing a device. However, it is also possible that offline or online recording of a device ID onto a control unit in a device is performed not only at a time of manufacturing the device, but also at any appropriate time.

Each of the devices transmits operation information indicating an operating state (operation state information) of the device to the server device at a predetermined time. More specifically, a control unit of each device generates operation information in which a user ID, a device ID, and a device state are associated with one another. Then, the communication unit 201 receives, via a network, the operation information generated by the control unit of the device. It should be noted that the control unit of each device may generate operation information at a plurality of predetermined times. It is possible to generate operation information at a time of transmission to the communication unit 201 of the server device 101a, or at a time independent from the time of transmitting the operation information.

Each device may transmit operation information at any time and at any frequency. For example, the device may transmit operation information regularly on every five minutes, while power is supplied. Likewise, each device may generate operation information at any time and at any frequency.

### (2) First Storage Unit

The first storage unit 202 holds pieces of operation information generated by the devices and received by the communication unit 201.

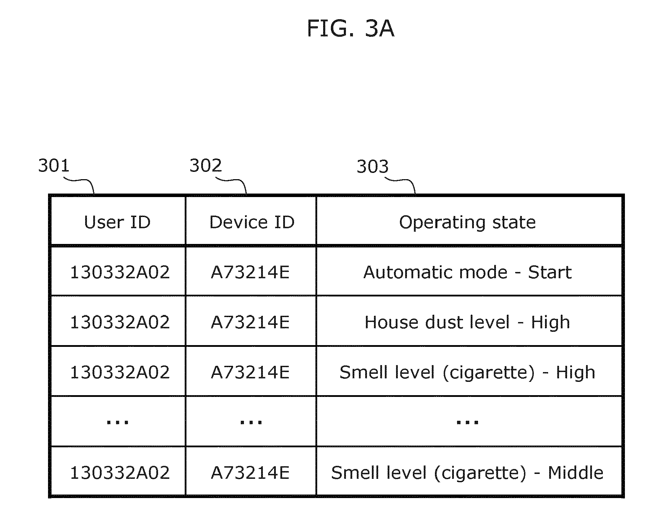

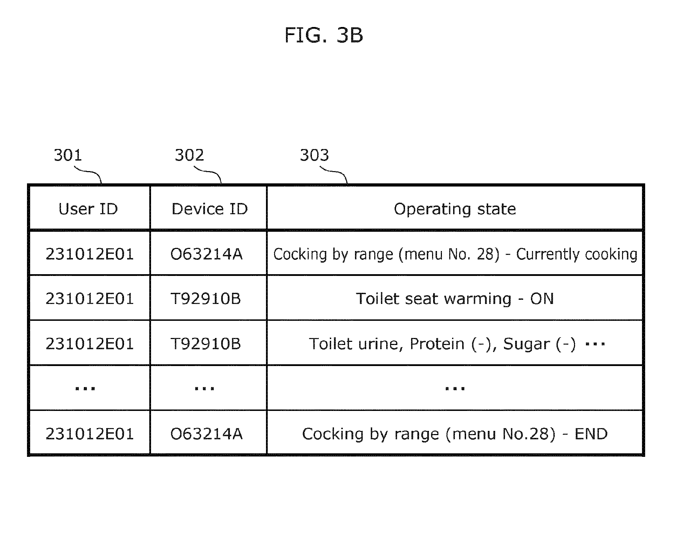

Each of FIGS. 3A and 3B is an example of data stored in the first storage unit 202 according to Embodiment 1 of the present invention. It is also possible to store time stamps, although they are not indicated in FIG. 3A and 3B. In other words, each operation information may be associated further with a time stamp indicating a time of generating the operation information.

As seen in FIG. 3A and FIG. 3B, the first storage unit 202 holds at least the user ID 301, the device ID 302, and the operating state 303.

In the examples of FIGS. 3A and 3B, an alphabet in the beginning of each device ID 302 indicates a type of the device, and a number following the alphabet indicates an importance level of information provided from the device. The importance level is classified to nine levels from "level 1 (low)" to "level 9 (high)". In the case of a device ID 302 "A73214E" in FIG. 3A, "A" in the beginning of the device ID indicates that the device is an air cleaner, and "7" following the alphabet indicates that an importance level of information provided from the air cleaner is "level 7". Likewise, in the case of a device ID 302 "O63214A" in FIG. 3B, "O" in the beginning indicates that the device is an oven, and "6" following the alphabet indicates that an importance level of information provided from the oven is "level 6".

Even devices of the same type have different importance levels of providing information, because different models of the same type have different sensors and the like. For example, while a sensor for detecting house dust is provided to every model of air cleaners, a smell sensor is provided only to upper models and not to medium and lower models. In this case, an importance level of information provided from an upper model is higher than an importance level of information provided from a medium or lower model, because the upper model has more kinds of sensors. Furthermore, even models having the same kind of sensor (for example, a smell sensor) have different importance levels of information provided from the devices depending on an accuracy of detection for each kind of smell. Therefore, it is possible that an importance level of information provided from a model having a smell sensor capable of detecting with high accuracy is set to higher than an importance level of information provided from a model having a smell sensor with low accuracy. In other words, it is possible that a model having more kinds of sensors is set to have a higher importance level of providing information, or that a model having a sensor with higher accuracy is set to have a higher importance level.

Furthermore, an operating state (operation state information) of a device includes not only an operating state of the device such as "Automatic mode - Start" in FIG. 3A, but also output values of sensors in the device, such as a "House dust level - High" in FIG. 3A and "Toilet urine, Protein (-), Sugar (-)" in FIG. 3B.

### (3) Second Storage Unit

The second storage unit 203 holds an operation pattern of each device. The operation pattern characterizes a tendency of user's behavior. In other words, the second storage unit 203 holds pieces of behavior tendency information in each of which an operating state of at least one of the devices is associated with a predetermined user behavior tendency.

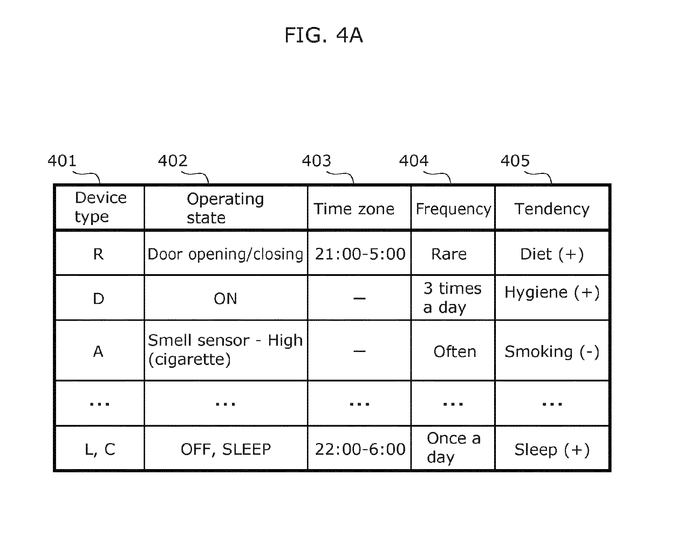

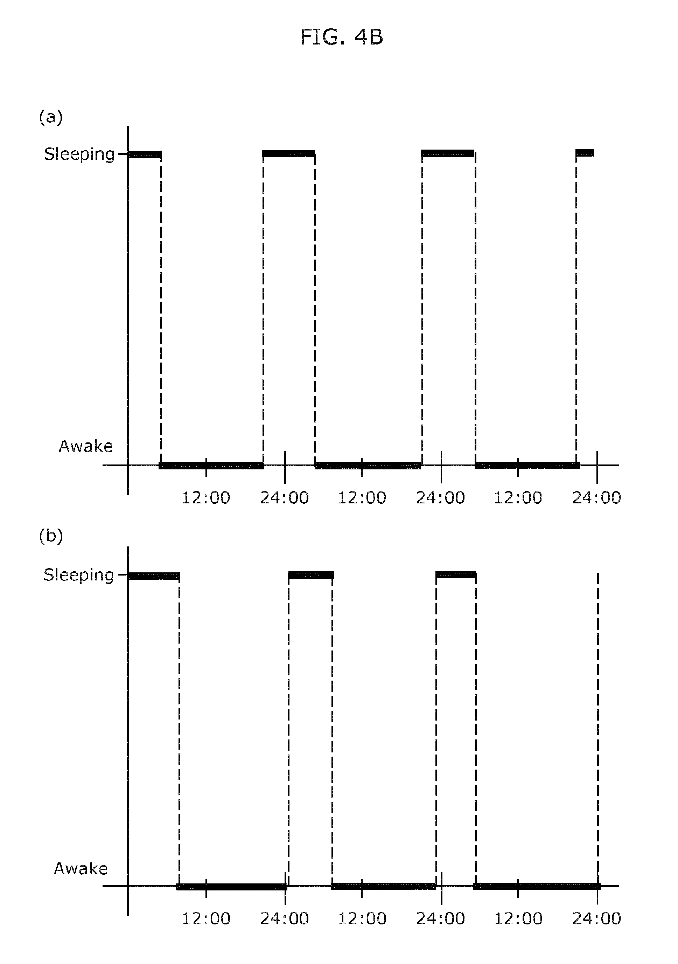

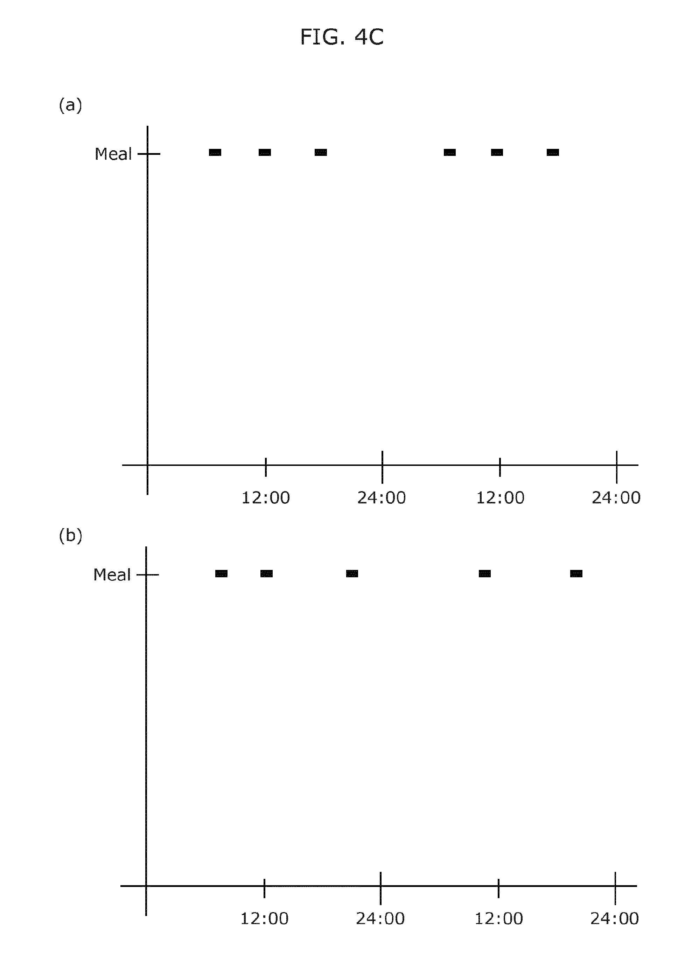

Each of FIG. 4A, (a) in FIG. 4B, and (a) in FIG. 4C is an example of data of pieces of behavior tendency information stored in the second storage unit 203 according to Embodiment 1 of the present invention. Each of (b) in FIG. 4B and (b) in FIG. 4C is an example of actual data of behavior information specified for the user based on the obtained operating state according to Embodiment 1 of the present invention.

As seen in FIG. 4A, the second storage unit 203 holds: an operation pattern that includes a device type 401, an operating state 402, a time zone 403, a frequency 404, and the like; and a tendency 405 of user's behavior which is associated with the operation pattern.

In FIG. 4A, as an example of "device type; operating state; time zone; frequency; tendency", there are indicated "R; Door opening/closing; 21:00 - 5:00; Rare; Diet (+)", "D; ON; -; 3 times a day; Hygiene (-)", "A; Smell sensor - High; -; Often; Smoking (+)", and "L, C; OFF, OFF; 22:00-6:00; Once a day; Sleep (+)". "R; Door opening/closing; 21:00-5:00; Rare; Diet (+)" means that "if a door of a refrigerator is rarely opened/closed during a period from 21:00 to 5:00, the diet habit is desirable". "D; ON; -; 3 times a day; Hygiene (-)" means that "if an electric tooth brush is turned ON three times a day, the hygienic habit is desirable". "A; Smell sensor - High; -; Often; Smoking (+)" means that "a smell sensor of an air cleaner shows a high value, and, in particular, if a frequency of detecting smell of cigarette is high, the user has a smoking habit and it is not desirable". "L, C; OFF, OFF; 22:00-6:00; Once a day; Sleep (+)" means that "if illumination in a bed room is OFF and a time zone in which a human detection sensor or the like of an air conditioner or the like detects that a user is sleeping is close to a time zone from 22:00 to 6:00, the sleeping habit is desirable". As "L, C; OFF, OFF; 22:00-6:00; Once a day; Sleep (+)", if there is a model in which an operation pattern indicating integrated operating states of a plurality of devices is associated with a user behavior tendency, the specification unit 204 can perform analysis with high accuracy. In other words, in the behavior tendency information, it is possible that an operating state obtained from one of the devices is associated with a predetermined user behavior tendency, or that operating states obtained from combined operating states of two or more devices among the devices is associated with the predetermined user behavior tendency.

It should be noted that although FIG. 4A shows, as the examples of the tendency, the diet habit, the hygienic habit, the smoking habit, and the sleeping habit, but, of course, the tendency is not limited to the examples. For example, the user behavior tendency may be information indicating user's character, such as "impatiens", "clumsy", or "calm".

Furthermore, as seen in (a) in FIG. 4B and (a) in FIG. 4C, the second storage unit 203 may hold, as behavior tendency information, a regularity model of desired behavior such as sleeping and eating/drinking. (a) in FIG. 4B shows that it is desirable to sleep for eight hours from 22:00 to 6:00. (a) in FIG 4C shows that it is desirable to eat three times a day in predetermined time zones.

Furthermore, the specification unit 204 compares the model in (a) in FIG. 4B and (a) in FIG. 4C to the user's actual data in (b) in FIG. 4B and (b) in FIG. 4C. If a correlation between (a) in FIG. 4B and (b) in FIG. 4B is high, it is possible to determine that the user has a desirable sleeping habit. If a correlation between (a) in FIG. 4C and (b) in FIG. 4C is high, it is possible to determine that the user has a desirable diet habit.

### (4) Specification Unit

The specification unit 204 reads the behavior tendency information from the second storage unit 203. Based on the readout behavior tendency information and the pieces of operation information stored in the first storage unit 202, the specification unit 204 searches the pieces of operation information stored in the first storage unit 202 for an operating state of at least one of the devices included in the behavior tendency information. As a result, the specification unit 204 specifies a predetermined user behavior tendency corresponding to the searched-out operation state(s).

It is also possible that the specification unit 204 specifies the user' behavior tendency based on timeliness of behavior with reference to the model as the behavior tendency information stored in the second storage unit 203 as seen in FIG. 4A, (a) in FIG. 4B, and (a) in FIG. 4C. In other words, the specification unit 204 may compare the pieces of operation information stored in the first storage unit 202 to the behavior tendency information to determine whether or not target user's behavior is performed at an appropriate time, so as to specify a user behavior tendency based on the determination result.

Therefore, it is possible to specify a user behavior tendency based on timeliness of behavior, such as a bedtime and a awake time, in other words, whether or not the user goes to bed early and gets up early, whether or not a time zone for meal is biased, or whether or not the user often has supper.

It is also possible that the specification unit 204 specifies a user behavior tendency based on regularity of behavior with reference to the model as the behavior tendency information stored in the second storage unit 203 as seen in FIG. 4A, (a) in FIG. 4B, and (a) in FIG. 4C. In other words, the specification unit 204 may compare the operation information stored in the first storage unit 202 to the behavior tendency information to determine whether or not the user's behavior is performed regularly, so as to specify a user behavior tendency based on the determination result.

Therefore, it is possible to specify a user behavior tendency based on regularity of behavior, such as whether or not a sleeping time is regular, or whether or not house cleaning or brushing teeth is performed in an appropriate cyclic way.

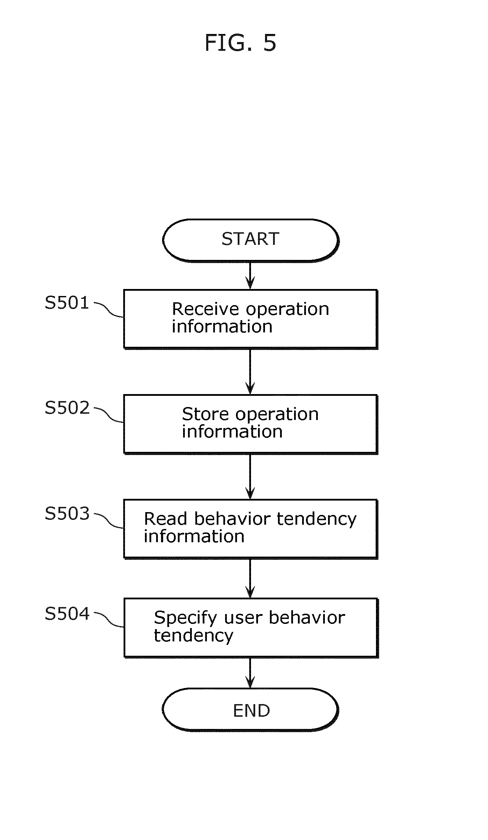

FIG. 5 is a flowchart of a method used in the server device 101a to specify a behavior tendency according to Embodiment 1 of the present invention.

The operation of the server device 101a will be described with reference to FIG. 5.

First, the communication unit 201 receives operation information from each of the devices 102 by communicating with the devices 102 (S501: receiving)

Next, the pieces of operation information received at Step S501 are stored into the first storage unit 202 (S502: storing).

The behavior tendency information is read from the second storage unit (S503: reading).

Then, based on the readout behavior tendency information and the pieces of operation information stored in the first storage unit 202, an operating state of at least one of devices which is included in the behavior tendency information is searched out from the pieces of operation information stored in the first storage unit 202, so as to specify a predetermined user behavior tendency corresponding to the searched-out operating state(s) (S504: specifying).

It should be noted that the user behavior tendency may be, for example, a risk of a disease (assessment value).

The second storage unit 203 may hold behavior tendency information in which an index indicates a user behavior tendency. The index is associated with each kind of operating states to increase or decrease an assessment value related to the user. Here, the specification unit 204 may compare the pieces of operation information stored in the first storage unit 202 with the behavior tendency information to calculate the assessment value to specify a user behavior tendency.

The index of the behavior tendency information may be, for example, an index for calculating a risk of a user's disease. In this case, the second storage unit 203 may hold an operating state of a device that increases or decreases a risk of each disease. Therefore, the specification unit 204 may calculate a risk of causing each disease based on a risk extracted by searching for the operating state of the device stored in the second storage unit 203. More specifically, the specification unit 204 may compare the pieces of operation information stored in the first storage unit 202 to the behavior tendency information to calculate a risk to specify a user behavior tendency. The disease may be a lifestyle-related disease.

The lifestyle-related disease is a collective term of diseases caused by a lifestyle habit. Lifestyle habits, such as a diet habit, an exercise habit, a sleeping habit, smoking, and alcohol drinking, are involved with onset and progress of diseases. Examples of lifestyle-related diseases are diabetes, cerebral stroke, heart disease, hyperlipidemia, hypertension, hyperuricemia, and obesity.

The lifestyle-related diseases include diseases for which a relationship between a lifestyle habit and the disease is apparent as presented below.
(a) diet habit: non-insulin-dependent diabetes mellitus, obesity, hyperlipidemia (except familial one), hyperuricemia, cardiovascular disease (except congenital one), colon cancer (except familial one), periodontal disease, etc.
(b) exercise habit: non-insulin-dependent diabetes mellitus, obesity, hyperlipidemia (except familial one), hypertension, etc.
(c) smoking: squamous cell cancer of a lung, cardiovascular disease (except congenital one), chronic bronchitis, emphysema, periodontal disease, etc.
(d) alcohol drinking: alcoholic hepatic disease, etc.

Onset of a disease is intricately related to various factors, such as "heritable factors" including abnormity of gene and aging, "external environment factors" including pathogens, harmful substances, accidents, and stressor, "lifestyle habit factors" including a diet habit and an exercise habit, and the like. Conventionally, it has been difficult to analyze a user behavior tendency by detecting behavior and lifestyle habit in home, in particularly, without using dedicated measurement devices.

The method for specifying a behavior tendency according to Embodiment 1 of the present invention calculates a risk of a lifestyle-related disease in the following manner.

If there is a refrigerator among the devices, the behavior tendency information may include an index for causing the specification unit 204 to calculate a the risk to be higher as the refrigerator is opened/closed at a higher frequency at night or before a bedtime. Furthermore, if there is a cleaner among the devices, the behavior tendency information may include an index for causing the specification unit 204 to calculate the risk to be higher as the cleaner is operated at a lower frequency and in a shorter time period. If there is an electric toothbrush among the devices, the behavior tendency information may include an index for causing the specification unit 204 to calculate the risk to be higher as the electric toothbrush is used at least at a lower frequency or in a shorter time period. If there is air cleaner among the devices, the behavior tendency information may include an index for causing the specification unit 204 to calculate the risk to be higher as a dust sensor in the air cleaner shows a greater value. The specification unit 204 may integrate these calculated risks to calculate a risk of a lifestyle-related disease.

### (Embodiment 2)

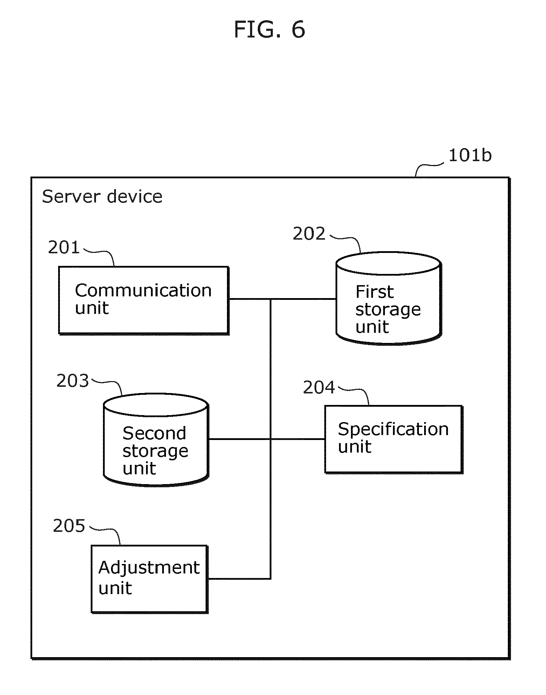

FIG. 6 is a diagram illustrating a structure of a server device according to Embodiment 2 of the present invention.

As illustrated in FIG. 6, a server device 101b includes the communication unit 201, the first storage unit 202, the second storage unit 203, the specification unit 204, and an adjustment unit 205. The server device 101b is an aspect of the server device 101 illustrated in FIG. 1.

More specifically, the server device 101b includes a microprocessor, a ROM, a RAM, a hard disk, and the like which are not illustrated in the figure. In each of the ROM, the RAM, and the hard disk, a computer program is recorded. When the microprocessor operates according to the computer program, the server device 101b functions.

It should be noted that each functional block, such as the communication unit 201, the first storage unit 202, the second storage unit 203, the specification unit 204, or the adjustment unit 205 may be implemented to software, to a combination of the software and an LSI that is an integrated circuit (namely, hardware), or to the LSI (namely, hardware).

The communication unit 201, the first storage unit 202, the second storage unit 203, and the specification unit 204 are the same as the corresponding units according to Embodiment 1, so that they are not described again below.

The adjustment unit 205 adjusts an assessment value based on at least one of: the number of devices providing pieces of operation information associated with the user information; and types of the devices. For example, a higher assessment value means more economic benefits for the user, which will be described later.

More specifically, the adjustment unit 205 may adjust the assessment value of the user which is calculated by the specification unit 204 to be lower as the number of the devices providing pieces of operation information associated with the user information is greater. Therefore, it is possible to provide the user with incentive to provide information. As a result, it is possible to induce the user to purchase the devices.

Furthermore, the adjustment unit 205 may adjust the assessment value of the user which is calculated by the specification unit 204 to be lower as the pieces of operation information associated with the user information include more operation information having a high importance level. Therefore, it is possible to provide the user with incentive to provide more operation information having a high importance level. As a result, it is possible to induce the user to purchase devices capable of providing operation information having a high importance level.

It should be noted that kinds of the operation information having a high importance level may include biological information such as urine data and blood sugar level data. For example, a toilet that measures urine data is disclosed in Japanese Patent No. 3565051 and the like. If urine data and the like can be obtained from such a toilet, it is possible to increase an accuracy of analysis by the specification unit 204. In particular, urine includes various components, such as sugar, protein, and occult blood, which have a concentration that varies depending on user's health condition. If urine data can be obtained, it is quite useful in calculating a risk of lifestyle-related disease such as diabetes. Likewise, blood sugar level data is also useful in calculating a risk of lifestyle-related disease. A blood sugar level measurement system is disclosed in Japanese Patent No. 5034720 and the like.

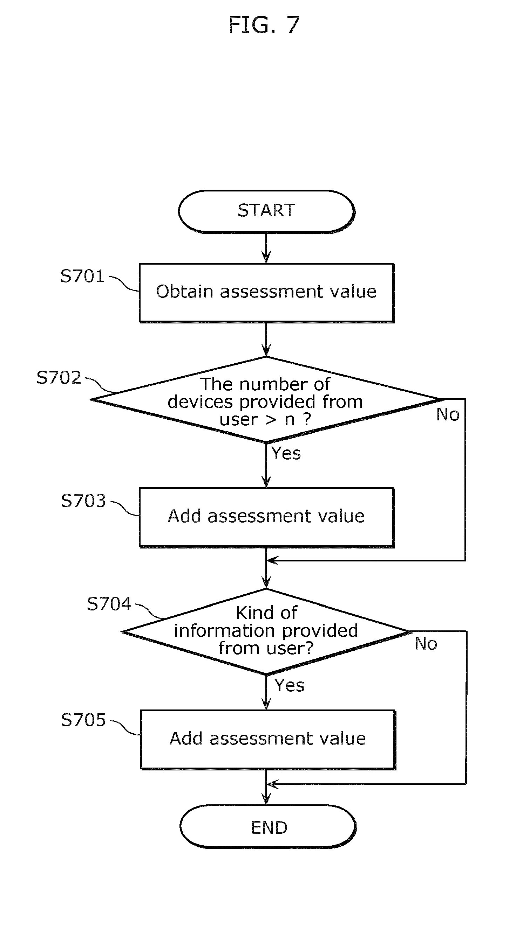

FIG. 7 is a flowchart of processing performed by the adjustment unit 205 according to Embodiment 2 of the present invention.

The processing of the adjustment unit 205 is described with reference to FIG. 7.

Here, for example, a risk is assessed by five levels from "level 5 (high)" to "level 1 (low)". It is assumed that a risk of "level 5" has a risk value ranging "from 100 to 81", that a risk of "level 4" has a risk value ranging "from 80 to 61", that a risk of "level 3" has a risk value ranging "from 60 to 41", that a risk of "level 2" has a risk value ranging "from 40 to 21", and a risk of "level 1" has a risk value ranging "from 20 to 0".

First, the adjustment unit 205 obtains a risk value R (assessment value) calculated by the specification unit 204 (Step S701). For example, a risk value R of a user K is assumed to be "68". Here, the level of the risk of the user K is classified to "level 4". Then, it is determined whether or not the number of pieces of information provided from the user is greater than a predetermined value "n" (Step S702). If the number of information provided from the user is greater than the predetermined value "n", the risk value R (assessment value) is decreased (Step S703). The number of the pieces of information provided from the user K is "12" that is greater than "n = 9". Therefore, a predetermined value "5" is subtracted from the risk value "R = 68" to obtain "R = 63". Here, the risk level is still classified to "level 4". Then, it is determined whether or not kinds of the pieces of information provided from the user include a predetermined kind (Step S704). If the kinds of the pieces of information provided from the user include the predetermined kind, the risk value R is decreased (Step S705). The user K provides information regarding a toilet which has "level 9" as an importance level indicated in FIG. 3B, and information regarding an air cleaner which has "level 7" as an importance level indicated in FIG. 3A. Therefore, the predetermined value "7" is subtracted from the risk value "R = 63" to obtain "R = 56". As a result, the risk level of the user K is lowered from "level 4" to "level 3" by one level. This influences compensation calculation described later. More specifically, if the risk level is lowered from "level 4" to "level 3" by one level, it is possible to provide the user with economic benefits such as cost down of insurance fee.

### (Embodiment 3)

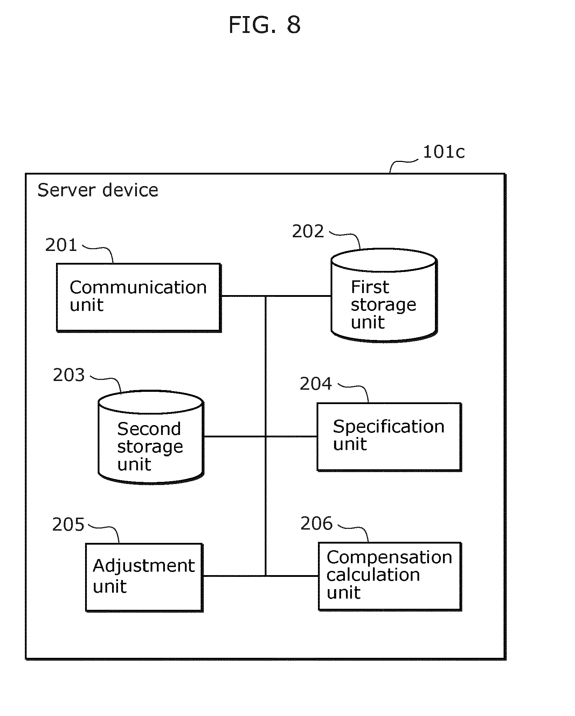

FIG. 8 is a diagram illustrating a structure of a server device 101c according to Embodiment 3 of the present invention.

As illustrated in FIG. 8, the server device 101c includes the communication unit 201, the first storage unit 202, the second storage unit 203, the specification unit 204, the adjustment unit 205, and a compensation calculation unit 206. It should be noted that the adjustment unit 205 is not essential. The server device 101c is an aspect of the server device 101 illustrated in FIG. 1.

The server device 101c includes a microprocessor, a ROM, a RAM, a hard disk, and the like which are not illustrated in the figure. In each of the ROM, the RAM, and the hard disk, a computer program is recorded. When the microprocessor operates according to the computer program, the server device 101c functions.

It should be noted that each functional block, such as the communication unit 201, the first storage unit 202, the second storage unit 203, the specification unit 204, the adjustment unit 205, and the compensation calculation unit 206 may be implemented to software, to a combination of the software and an LSI that is an integrated circuit (namely, hardware), or to the LSI (namely, hardware).

The communication unit 201, the first storage unit 202, the second storage unit 203, and the specification unit 204 are the same as the corresponding units according to Embodiment 1, so that they are not described again below.

The adjustment unit 205 is the same as the corresponding unit in Embodiment 2, so that it is not described again below.

The compensation calculation unit 206 calculates economic compensation for an assessment value, based on the assessment value calculated by the specification unit 204. More specifically, the economic compensation may be a point given to the user, or a price of a product or service which is purchased by or provided to the user. The price may be a price of insurance product, namely, an insurance fee. More specifically, the compensation calculation unit 206 may calculate an economic compensation for an assessment value with reference to a table in which an economic compensation is predetermined for an assessment value, or by using a relationship formula between the predetermined assessment value and the economic compensation. In other words, the compensation calculation unit 206 calculates the economic compensation based on a relationship between the predetermined assessment value and the economic compensation.

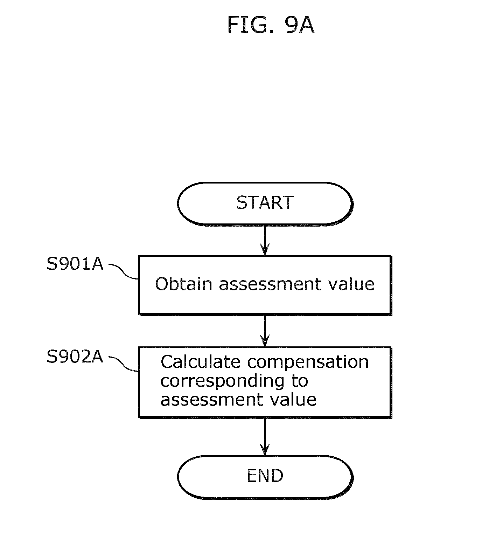

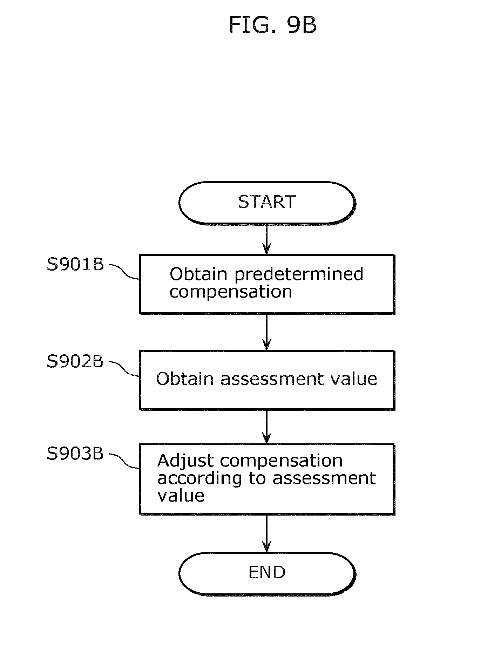

Each of FIGS. 9A and 9B is a flowchart of processing performed by the compensation calculation unit 206 according to Embodiment 3 of the present invention.

The processing of the compensation calculation unit 206 is described with reference to FIGS. 9A and 9B.

As illustrated in FIG. 9A, first, the compensation calculation unit 206 obtains a risk calculated by the specification unit 204 as an assessment value, or an assessment value (risk) adjusted by the adjustment unit 205 (Step S901A). Then, with reference to the table in which a value is predetermined for each assessment value, a compensation corresponding to the obtained assessment value is calculated (Step S902A).

Or, as seen in FIG. 9B, the compensation calculation unit 206 first obtains a predetermined compensation (Step S901B). Then, the assessment value calculated by the specification unit 204 or the assessment value adjusted by the adjustment unit 205 is obtained (Step S902B), and the compensation is adjusted by the obtained assessment value (Step S903B).

Meanwhile, there are various insurance products, such as life insurance, medical insurance, cancer insurance, lifestyle-related disease insurance, and pet insurance.

An insurance fee of conventional life insurance is calculated based on three assumed rates that are an assumed mortality rate, an assumed interest rate, and an assumed operating expense rate. The assumed rate is an experience assumption assumed in contracting the insurance. Based on past statistics, the number of fatality (survivors) of each sex and age is predicted, and thereby an amount required for future payments such as insurance payment is calculated. The number of fatality used in the prediction is the assumed mortality rate. Furthermore, the life insurance companies discount expected certain yield of investment management from the insurance fee. The discount rate is called an assumed interest rate. Furthermore, the life insurance companies expect miscellaneous expense necessary for business operations, such as contract execution, insurance fee reception, and contract maintenance management. The miscellaneous expense is called an assumed operating expense rate. It should be noted that these assumed rates vary depending on types of insurance or time of contract.

As described above, conventionally, an insurance fee is calculated based on the situation in the insurance contract. However, among diseases, many diseases progress slowly, such as lifestyle-related diseases. Furthermore, many diseases do not show subjective symptom although they should be found and treated in the early stage. Therefore, it is desirable to calculate and revise an insurance fee based on daily lifestyle habit.

The data analysis system according to the present invention calculates a risk of a lifestyle-related disease at the time, based on pieces of information obtained from various kinds of devices or systems, such as home appliances including: a television set, an air conditioner, a refrigerator, a washing machine, a cleaner, an air cleaner, an electric toothbrush, and a dryer; information terminals including a personal computer, a mobile telephone, a smartphone, and a tablet; sensor devices including a blood sugar level sensor device; a toilet: a bathroom; a mirror; a lighting device; and the like. The data analysis system is capable of calculating and revising an insurance fee according to the calculated risk. As a result, an insurance company is able to assume a risk with higher accuracy than that of the conventional techniques. On the other hand, if the user improves his/her lifestyle habit, the user has advantages, for example, that the assessment value is decreased to lower the insurance fee. Therefore, it is expected to increase user's motivation for improving a lifestyle habit.

As described above, it is possible to analyze a tendency of user's behavior based on daily life behavior.

It should be noted that it has been described in the above embodiment that the example is an insurance related to user's health. However, the present embodiment may be applied to an insurance not related to user's health, such as automobile insurance and fire insurance. For example, in the case of automobile insurance, the present invention can be applied if a tendency of user's behavior which is considered to be likely to cause automobile accidents, such as user's characters "impatiens" or "bad-tempered", is assessed as an assessment value.

Although the data analysis system according to one or more aspects of the present invention has been described based on the above embodiments and variations, the present invention is not limited to the embodiments and variations. Those skilled in the art will be readily appreciated that various modifications and combinations of the structural elements in the different embodiments and variations are possible without materially departing from the novel teachings and advantages of the present invention. Accordingly, all such modifications and combinations are intended to be included within the scope of the present invention.

Furthermore, the present invention may have the following variations.
(1) Each of the above devices may be a computer system including a microprocessor, a Read Only Memory (ROM), a Random Access Memory (RAM), a hard disk unit, a display unit, a keyboard, a mouse, and the like. The RAM or the hard disk unit holds a computer program. The microprocessor operates according to the computer program, thereby causing each of the devices to perform its functions. Here, the computer program consists of combinations of instruction codes for issuing instructions to the computer to execute predetermined functions. It should be noted that each of the devices is not limited to a computer system including a microprocessor, a Read Only Memory (ROM), a Random Access Memory (RAM), a hard disk unit, a display unit, a keyboard, a mouse, and the like, but to a computer system including a part of them.
(2) It should be noted that a part or all of the structural elements included in each of the devices may be implemented into a single Large Scale Integration (LSI). The system LSI is a super multi-function LSI that is a single chip into which a plurality of structural elements are integrated. More specifically, the system LSI is a computer system including a microprocessor, a ROM, a RAM, and the like. The RAM holds a computer program. The microprocessor operates according to the computer program to cause the system LSI to perform its functions.

Here, the integrated circuit is referred to as a LSI, but the integrated circuit can be called an IC, a system LSI, a super LSI or an ultra LSI depending on their degrees of integration. The technique of integrated circuit is not limited to the LSI, and it may be implemented as a dedicated circuit or a general-purpose processor. It is also possible to use a Field Programmable Gate Array (FPGA) that can be programmed after manufacturing the LSI, or a reconfigurable processor in which connection and setting of circuit cells inside the LSI can be reconfigured.

Furthermore, if due to the progress of semiconductor technologies or their derivations, new technologies for integrated circuits appear to be replaced with the LSIs, it is, of course, possible to use such technologies to implement the functional blocks as an integrated circuit. Biotechnology and the like can be applied to the above implementation.
(3) It should also be noted that a part or all of the structural elements included in each of the devices may be implemented into an Integrated Circuit (IC) card or a single module which is attachable to and removable from the device. The IC card or the module is a computer system including a microprocessor, a ROM, a RAM, and the like. The IC card or the module may include the above-described super multi-function LSI. The microprocessor operates according to the computer program to cause the IC card or the module to perform its functions. The IC card or the module may have tamper resistance.
(4) It should also be noted that the present invention may be the above-described method. The present invention may be a computer program causing a computer to execute the method, or digital signals indicating the computer program.

It should also be noted that the present invention may be a computer-readable recording medium on which the computer program or the digital signals are recorded. Examples of the computer-readable recording medium are a flexible disk, a hard disk, a Compact Disc (CD)-ROM, a magnetooptic disk (MO), a Digital Versatile Disc (DVD), a DVD-ROM, a DVD-RAM, a BD (Blu-ray^{®} Disc), and a semiconductor memory. The present invention may be digital signals recorded on the recording medium.

It should also be noted in the present invention that the computer program or the digital signals may be transmitted via an electric communication line, a wired or wireless communication line, a network represented by the Internet, data broadcasting, and the like.

It should also be noted that the present invention may be a computer system including a microprocessor operating according to the computer program and a memory storing the computer program.

It should also be noted that the program or the digital signals may be recorded onto the recording medium to be transferred, or may be transmitted via a network or the like, so that the program or the digital signals can be executed by a different independent computer system.
(5) It should also be noted that the above-described embodiments and their variations may be combined.

The disclosed embodiments are merely exemplary and do not limit the present invention. The scope of the present invention is indicated not by the above description but by the appended claims. Accordingly, all modifications are intended to be included within the same meanings and the scope of the claims.

### [Industrial Applicability]

The data analysis system according to the present invention is useful as a system or the like that integrates various pieces of information and thereby analyzes a tendency of user's behavior.

### [Reference Signs List]

- 101, 101a to 101c: server device
- 102: device
- 201: communication unit
- 202: first storage unit
- 203: second storage unit
- 204: specification unit
- 205: adjustment unit
- 206: compensation calculation unit
- 301: user ID
- 302: device ID
- 303: operating state
- 401: device type
- 402: operating state
- 403: time zone
- 404: frequency
- 405: tendency

## Claims

1. A method for specifying a behavior tendency, the method comprising:
receiving, through communication, pieces of operation information each including (a) operating state information of a corresponding one of devices and (b) user information for identifying a user, in association with each other;
storing the pieces of the operation information received in the receiving into a first storage unit;
reading at least one piece of behavior tendency information from a second storage unit, the at least one piece of the behavior tendency information each including, in association with each other, (c) operating state information of at least one of the devices and (d) a predetermined user behavior tendency of the user; and
specifying, based on the at least one piece of the behavior tendency information read in the reading and the pieces of the operation information stored in the first storage unit, the predetermined user behavior tendency which corresponds to the operating state information of the at least one of the devices, by searching the pieces of the operation information for the operating state information of the at least one of the devices which is included in the at least one piece of the behavior tendency information.

2. The method according to Claim 1,
wherein each of the pieces of the operation information includes the operating state information that is generated at a corresponding time for the corresponding one of the devices, and
in the specifying, the pieces of the operation information stored in the first storage unit are compared to the at least one piece of the behavior tendency information to determine whether or not behavior of the user is performed at an appropriate time, and the predetermined user behavior tendency is specified based on a result of the determining.

3. The method according to Claim 1,
wherein each of the pieces of the operation information includes the operating state information that is generated at a corresponding time for the corresponding one of the devices, and
in the specifying, the pieces of the operation information stored in the first storage unit are compared to the at least one piece of the behavior tendency information to determine whether or not behavior of the user is performed regularly, and the predetermined user behavior tendency is specified based on a result of the determining.

4. The method according to any one of Claims 1 to 3,
wherein in the reading, the at least one piece of the behavior tendency information includes an index that indicates the predetermined user behavior tendency and that is associated with each kind of the operating state information to increase or decrease an assessment value related to the user, and
in the specifying, the predetermined user behavior tendency is specified, by comparing the pieces of the operation information stored in the first storage unit to the at least one piece of the behavior tendency information to calculate the assessment value.

5. The method according to Claim 4, further comprising
adjusting the assessment value based on at least one of (a) a total number of the devices providing the pieces of the operation information associated with the user information and (b) types of the devices.

6. The method according to Claim 5,
wherein in the adjusting, the assessment value of the user which is calculated in the specifying is adjusted to be lower as the total number of the devices providing the pieces of the operation information associated with the user information is greater.

7. The method according to Claim 5 or 6,
wherein in the adjusting, the assessment value of the user which is calculated in the specifying is adjusted to be lower as the pieces of the operation information associated with the user information include more pieces of information having a high importance level.

8. The method according to Claim 7,
wherein a kind of the information having the high importance level is urine data.

9. The method according to Claim 7 or 8,
wherein a kind of the information having the high importance level is blood sugar level data.

10. The method according to any one of Claims 4 to 9,
wherein the index in the at least one piece of the behavior tendency information is for calculating a risk of a disease of the user, and
in the specifying, the predetermined user behavior tendency is specified, by comparing the pieces of the operation information stored in the first storage unit to the at least one piece of the behavior tendency information to calculate the risk.

11. The method according to Claim 10,
wherein the disease is a lifestyle-related disease.

12. The method according to Claim 10 or 11,
wherein the devices include a refrigerator, and
the index in the at least one piece of the behavior tendency information is for calculating the risk of the user to be higher as the refrigerator is opened and closed at a higher frequency at night or before a bedtime.

13. The method according to any one of Claims 10 to 12,
wherein the devices include a cleaner, and
the index in the at least one piece of the behavior tendency information is for calculating the risk of the user to be higher as the cleaner is operated at least one of at a lower frequency and in a shorter time period.

14. The method according to any one of Claims 10 to 13,
wherein the devices include an electric toothbrush, and
the index in the at least one piece of the behavior tendency information is for calculating the risk of the user to be higher as the electric toothbrush is used at least one of at a lower frequency and in a shorter time period.

15. The method according to any one of Claims 10 to 14,
wherein the devices include an air cleaner, and
the index in the at least one piece of the behavior tendency information is for calculating the risk of the user to be higher as a dust sensor in the air cleaner shows a greater value.

16. The method according to any one of Claims 4 to 15, further comprising
calculating an economic compensation for the assessment value calculated in the specifying, based on the assessment value.

17. The method according to Claim 16,
wherein in the calculating of the economic compensation, a point to be given to the user is calculated as the economic compensation.

18. The method according to Claim 16,
wherein in the calculating of the economic compensation, an insurance fee of insurance for the user is calculated as the economic compensation.

19. A system for specifying a behavior tendency, the system comprising:
a communication unit configured to receive pieces of operation information each including (a) operating state information of a corresponding one of devices including a home appliance and (b) user information for identifying a user, in association with each other;
a first storage unit configured to hold the pieces of the operation information received by the communication unit;
a second storage unit configured to hold at least one piece of behavior tendency information, the at least one piece of the behavior tendency information each including, in association with each other, (c) operating state information of at least one of the devices and (d) a predetermined user behavior tendency of the user; and
a specification unit configured to specify the predetermined user behavior tendency which corresponds to the operating state information of the at least one of the devices, by (i) reading the at least one piece of the behavior tendency information from the second storage unit and the pieces of the operation information from the first storage unit, and (ii) searching the pieces of the operation information in the first storage unit for the operating state information of the at least one of the devices which is included in the at least one piece of the behavior tendency information.
